# EUROPEAN PATENT SPECIFICATION

(11) **EP 1 722 819 B1**
(45) Date of publication and mention of the grant of the patent: **26.12.2007**
(21) Application number: 05727232.0
(22) Date of filing: 11.03.2005
(51) Int. Cl.: A61K 47/12, A61K 39/12, A61K 39/02, A61K 38/02

(54) **METHOD FOR SOLUBILISING PEPTIDE MIXTURES**
VERFAHREN ZUR SOLUBILISIERUNG VON PEPTID-MISCHUNGEN
PROCEDE DE SOLUBILISATION DE MELANGES DE PEPTIDES

(30) Priority: 12.03.2004 EP 04450061
(43) Date of publication of application: 22.11.2006
(73) Proprietor: Intercell AG, 1030 Vienna (AT)
(72) Inventor: ZAUNER, Wolfgang, A-1030 Wien (AT); KRITSCH, Constantia, A-7100 Neusiedl (AT); HEINRICH-CSEH, Christa, A-1170 Vienna (AT); BERGER, Agnes, A-2540 Bad Vöslau (AT)
(74) Representative: Sonn & Partner Patentanwälte
(86) International application number: PCT/EP2005/002583
(87) International publication number: WO 2005/094891

(56) References cited:
- EP-A- 0 508 435
- EP-A- 0 998 942
- EP-A- 1 254 666
- WO-A-20/05004910
- NENCI ET AL: "Structural properties of trimers and tetramers of ribonuclease A" PROTEIN SCIENCE, vol. 10, 2001, pages 2017-2027, XP002359794

### SEQUENCE LISTING

<110> Intercell AG
<120> Method for solubilising Peptide Mixtures
<130> R 45101
<140> PCT/EP2005/002583
   <141> 2005-03-11
<150> EP 04450061.9
   <151> 2004-03-12
<160> 47
<170> PatentIn version 3.3
<210> 1
   <211> 20
   <212> PRT
   <213> Artificial
<220>
   <223> Artificial Peptide
<400> 1
   Ile Asp Glu Leu Lys Thr Asn Ser Ser Leu Leu Thr Ser Ile Leu Thr
   1               5                   10                  15
   Tyr His Val Val
               20
<210> 2
   <211> 17
   <212> PRT
   <213> Artificial
<220>
   <223> Artificial Peptide
<400> 2
   Thr Gly Ser Gly Ala Gly Ile Ala Gln Ala Ala Ala Gly Thr Val Asn
   1               5                   10                  15
   Ile
<210> 3
   <211> 20
   <212> PRT
   <213> Artificial
<220>
   <223> Artificial Peptide
<400> 3
   Val Ser Asp Leu Lys Ser Ser Thr Ala Val Ile Pro Gly Tyr Pro Val
   1               5                   10                  15
   Ala Gly Gln Val
               20
<210> 4
   <211> 17
   <212> PRT
   <213> Artificial
<220>
   <223> Artificial Peptide
<400> 4
   Asn Phe Leu Leu Pro Asp Ala Gln Ala Ala Ala Ala Gly Phe Ala Ser
   1               5                   10                  15
   Lys
<210> 5
   <211> 20
   <212> PRT
   <213> Artificial
<220>
   <223> Artificial Peptide
<400> 5
   Tyr Asn Ile Asn Ile Ser Leu Pro Ser Tyr Tyr Pro Asp Gln Lys Ser
   1               5                   10                  15
   Leu Glu Asn Tyr
               20
<210> 6
   <211> 22
   <212> PRT
   <213> Artificial
<220>
   <223> Artificial Peptide
<400> 6
   Lys Phe Pro Gly Gly Gly Gln Ile Val Gly Gly Val Tyr Leu Leu Pro
   1 5 10 15
   Arg Arg Gly Pro Arg Leu
   20
<210> 7
   <211> 14
   <212> PRT
   <213> Artificial
<220>
   <223> Artificial Peptide
<400> 7
   Gly Tyr Lys Val Leu Val Leu Asn Pro Ser Val Ala Ala Thr
   1 5 10
<210> 8
   <211> 9
   <212> PRT
   <213> Artificial
<220>
   <223> Artificial Peptide
<400> 8
   Asp Leu Met Gly Tyr Ile Pro Ala Val
   1 5
<210> 9
   <211> 9
   <212> PRT
   <213> Artificial
<220>
   <223> Artificial Peptide
<400> 9
   Cys Ile Asn Gly Val Cys Trp Thr Val
   1 5
<210> 10
   <211> 23
   <212> PRT
   <213> Artificial
<220>
   <223> Artificial Peptide
<400> 10
   His Met Trp Asn Phe Ile Ser Gly Ile Gln Tyr Leu Ala Gly Leu Ser
   1 5 10 15
   Thr Leu Pro Gly Asn Pro Ala
   20
<210> 11
   <211> 29
   <212> PRT
   <213> Artificial
<220>
   <223> Artificial Peptide
<400> 11
   Lys Phe Pro Gly Gly Gly Gln Ile Val Gly Gly Val Tyr Leu Leu Pro
   1 5 10 15
   Arg Arg Gly Pro Arg Leu Gly Val Arg Ala Thr Arg Lys
   20 25
<210> 12
   <211> 19
   <212> PRT
   <213> Artificial
<220>
   <223> Artificial Peptide
<400> 12
   Leu Glu Asp Arg Asp Arg Ser Glu Leu Ser Pro Leu Leu Leu Ser Thr
   1 5 10 15
   Thr Glu Trp
<210> 13
   <211> 20
   <212> PRT
   <213> Artificial
<220>
   <223> Artificial Peptide
<400> 13
   Asp Tyr Pro Tyr Arg Leu Trp His Tyr Pro Cys Thr Val Asn Phe Thr
   1 5 10 15
   Ile Phe Lys Val
   20
<210> 14
   <211> 16
   <212> PRT
   <213> Artificial
<220>
   <223> Artificial Peptide
<400> 14
   Ala Ala Trp Tyr Glu Leu Thr Pro Ala Glu Thr Thr Val Arg Leu Arg
   1 5 10 15
<210> 15
   <211> 21
   <212> PRT
   <213> Artificial
<220>
   <223> Artificial Peptide
<400> 15
   Tyr Leu Val Ala Tyr Gln Ala Thr Val Cys Ala Arg Ala Gln Ala Pro
   1 5 10 15
   Pro Pro Ser Trp Asp
   20
<210> 16
   <211> 12
   <212> PRT
   <213> Artificial
<220>
   <223> Artificial Peptide
<400> 16
   Thr Ala Tyr Ser Gln Gln Thr Arg Gly Leu Leu Gly
   1 5 10
<210> 17
   <211> 26
   <212> PRT
   <213> Artificial
<220>
   <223> Artificial Peptide
<400> 17
   Ile Gly Leu Gly Lys Val Leu Val Asp Ile Leu Ala Gly Tyr Gly Ala
   1               5                   10                  15
   Gly Val Ala Gly Ala Leu Val Ala Phe Lys
               20                  25
<210> 18
   <211> 13
   <212> PRT
   <213> Artificial
<220>
   <223> Artificial Peptide
<400> 18
   Ser Met Ser Tyr Thr Trp Thr Gly Ala Leu Ile Thr Pro
   1               5                   10
<210> 19
   <211> 15
   <212> PRT
   <213> Artificial
<220>
   <223> Artificial Peptide
<400> 19
   Thr Thr Leu Leu Phe Asn Ile Leu Gly Gly Trp Val Ala Ala Gln
   1               5                   10                  15
<210> 20
   <211> 9
   <212> PRT
   <213> Artificial
<220>
   <223> Artificial Peptide
<400> 20
   Thr Val Asn Tyr Thr Ile Phe Lys Ile
   1 5
<210> 21
   <211> 20
   <212> PRT
   <213> Artificial
<220>
   <223> Artificial Peptide
<400> 21
   Met Trp Asn Phe Ile Ser Gly Ile Gln Tyr Leu Ala Gly Leu Ser Thr
   1 5 10 15
   Leu Pro Gly Asn
   20
<210> 22
   <211> 10
   <212> PRT
   <213> Artificial
<220>
   <223> Artificial Peptide
<400> 22
   Tyr Leu Leu Pro Arg Arg Gly Pro Arg Leu
   1 5 10
<210> 23
   <211> 23
   <212> PRT
   <213> Artificial
<220>
   <223> Artificial Peptide
<400> 23
   Gly Trp Arg Leu Leu Ala Pro Ile Thr Ala Tyr Ser Gln Gln Thr Arg
   1 5 10 15
   Gly Leu Leu Gly Cys Ile Val
   20
<210> 24
   <211> 18
   <212> PRT
   <213> Artificial
<220>
   <223> Artificial Peptide
<400> 24
   Val Val Cys Cys Ser Met Ser Tyr Thr Trp Thr Gly Ala Leu Ile Thr
   1 5 10 15
   Pro Cys
<210> 25
   <211> 15
   <212> PRT
   <213> Artificial
<220>
   <223> Artificial Peptide
<400> 25
   Asp Tyr Pro Tyr Arg Leu Trp His Tyr Pro Cys Thr Val Asn Phe
   1 5 10 15
<210> 26
   <211> 15
   <212> PRT
   <213> Artificial
<220>
   <223> Artificial Peptide
<400> 26
   Ala Gly Ala Ala Trp Tyr Glu Leu Thr Pro Ala Glu Thr Thr Val
   1 5 10 15
<210> 27
   <211> 15
   <212> PRT
   <213> Artificial
<220>
   <223> Artificial Peptide
<400> 27
   Gly Ser Ile Gly Leu Gly Lys Val Leu Val Asp Ile Leu Ala Gly
   1 5 10 15
<210> 28
   <211> 15
   <212> PRT
   <213> Artificial
<220>
   <223> Artificial Peptide
<400> 28
   Leu Ala Gly Tyr Gly Ala Gly Val Ala Gly Ala Leu Val Ala Phe
   1 5 10 15
<210> 29
   <211> 15
   <212> PRT
   <213> Artificial
<220>
   <223> Artificial Peptide
<400> 29
   Leu Trp His Tyr Pro Cys Thr Val Asn Phe Thr Ile Phe Lys Val
   1 5 10 15
<210> 30
   <211> 11
   <212> PRT
   <213> Artificial
<220>
   <223> Artificial Peptide
<400> 30
   Arg Met Tyr Val Gly Gly Val Glu His Arg Leu
   1 5 10
<210> 31
   <211> 13
   <212> PRT
   <213> Artificial
<220>
   <223> Artificial Peptide
<400> 31
   Asp Leu Met Gly Tyr Ile Pro Leu Val Gly Ala Pro Leu
   1               5                   10
<210> 32
   <211> 8
   <212> PRT
   <213> Artificial
<220>
   <223> Artificial Peptide
<400> 32
   Thr Ile Asn Tyr Thr Ile Phe Lys
   1               5
<210> 33
   <211> 32
   <212> PRT
   <213> Artificial
<220>
   <223> Artificial Peptide
<400> 33
   Val Asp Tyr Pro Tyr Arg Leu Trp His Tyr Pro Cys Thr Val Asn Phe
   1               5                   10                  15
   Thr Ile Phe Lys Val Arg Met Tyr Val Gly Gly Val Glu His Arg Leu
               20                  25                  30
<210> 34
   <211> 20
   <212> PRT
   <213> Artificial
<220>
   <223> Artificial Peptide
<400> 34
   Asp Tyr Pro Tyr Arg Leu Trp His Tyr Pro Cys Thr Val Asn Phe Thr
   1               5                   10                  15
   Ile Phe Lys Ile
               20
<210> 35
   <211> 32
   <212> PRT
   <213> Artificial
<220>
   <223> Artificial Peptide
<400> 35
   Val Asp Tyr Pro Tyr Arg Leu Trp His Tyr Pro Cys Thr Val Asn Tyr
   1               5                   10                  15
   Thr Ile Phe Lys Ile Arg Met Tyr Val Gly Gly Val Glu His Arg Leu
               20                  25                  30
<210> 36
   <211> 20
   <212> PRT
   <213> Artificial
<220>
   <223> Artificial Peptide
<400> 36
   Asp Tyr Pro Tyr Arg Leu Trp His Tyr Pro Cys Thr Val Asn Tyr Thr
   1               5                   10                  15
   Ile Phe Lys Ile
               20
<210> 37
   <211> 19
   <212> PRT
   <213> Artificial
<220>
   <223> Artificial Peptide
<400> 37
   Ala Tyr Ala Ala Gln Gly Tyr Lys Val Leu Val Leu Asn Pro Ser Val
   1               5                   10                  15
   Ala Ala Thr
<210> 38
   <211> 25
   <212> PRT
   <213> Artificial
<220>
   <223> Artificial Peptide
<400> 38
   Gly Glu Val Gln Val Val Ser Thr Ala Thr Gln Ser Phe Leu Ala Thr
   1 5 10 15
   Cys Ile Asn Gly Val Cys Trp Thr Val
   20 25
<210> 39
   <211> 15
   <212> PRT
   <213> Artificial
<220>
   <223> Artificial Peptide
<400> 39
   Thr Ala Tyr Ser Gln Gln Thr Arg Gly Leu Leu Gly Cys Ile Val
   1 5 10 15
<210> 40
   <211> 17
   <212> PRT
   <213> Artificial
<220>
   <223> Artificial Peptide
<400> 40
   Met Ser Thr Asn Pro Lys Pro Gln Arg Lys Thr Lys Arg Asn Thr Asn
   1 5 10 15
   Arg
<210> 41
   <211> 21
   <212> PRT
   <213> Artificial
<220>
   <223> Artificial Peptide
<400> 41
   Leu Ile Asn Thr Asn Gly Ser Trp His Ile Asn Arg Thr Ala Leu Asn
   1 5 10 15
   Cys Asn Asp Ser Leu
   20
<210> 42
   <211> 15
   <212> PRT
   <213> Artificial
<220>
   <223> Artificial Peptide
<400> 42
   Thr Thr Ile Leu Gly Ile Gly Thr Val Leu Asp Gln Ala Glu Thr
   1 5 10 15
<210> 43
   <211> 18
   <212> PRT
   <213> Artificial
<220>
   <223> Artificial Peptide
<400> 43
   Phe Asp Ser Ser Val Leu Cys Glu Cys Tyr Asp Ala Gly Ala Ala Trp
   1 5 10 15
   Tyr Glu
<210> 44
   <211> 20
   <212> PRT
   <213> Artificial
<220>
   <223> Artificial Peptide
<400> 44
   Ala Arg Leu Ile Val Phe Pro Asp Leu Gly Val Arg Val Cys Glu Lys
   1 5 10 15
   Met Ala Leu Tyr
   20
<210> 45
   <211> 15
   <212> PRT
   <213> Artificial
<220>
   <223> Artificial Peptide
<400> 45
   Ala Phe Cys Ser Ala Met Tyr Val Gly Asp Leu Cys Gly Ser Val
   1               5                   10                  15
<210> 46
   <211> 15
   <212> PRT
   <213> Artificial
<220>
   <223> Artificial Peptide
<400> 46
   Gly Val Leu Phe Gly Leu Ala Tyr Phe Ser Met Val Gly Asn Trp
   1               5                   10                  15
<210> 47
   <211> 15
   <212> PRT
   <213> Artificial
<220>
   <223> Artificial Peptide
<400> 47
   Thr Arg Val Pro Tyr Phe Val Arg Ala Gln Gly Leu Ile Arg Ala
   1               5                   10                  15

## Description

The present invention relates to a method for solubilising peptide mixtures.

The formulation of peptides and peptide mixtures for pharmaceutical use is in many cases very challenging. Ideally, a formulation should be stable at room temperature or at least at 4°C for prolonged time (up to two years). Especially this long-term stability is difficult to achieve in solution since a lot of reactions can take place at the side chain functional groups of amino acids, such as oxidation (tryptophane, methionine, cysteine), deamidation (glutamine, asparagine), and racemisation. In addition depending on the pH of the final formulation hydrolysis of the peptide backbone can also occur. Therefore freeze-drying is a preferred way of obtaining stable peptide formulations.

The solubilisation of single peptides usually represents in the laboratory practice a minor problem. Whereas hydrophilic peptides are usually dissolved in water or buffered aqueous solutions, hydrophobic peptides are dissolved in solutions containing in many cases at least one component of an organic solvent. The solubilisation of a mixture of chemically different peptides is much more complicated due to the presence of a variety of hydrophilic (water soluble) and hydrophobic (water insoluble) peptides.

Therefore the challenges for a pharmaceutical formulation containing a peptide mixture are several-fold. First of all a solvent has to be found that allows the solubilization of all peptides at the required concentration. Furthermore the solvent has to be relatively non-toxic, so that traces of residual solvent in the product do not prevent pharmaceutical use. Finally to prolong the shelf life of a peptide/protein mixture the composition should be lyophilisable (mixtures of e.g. water and DMSO cannot be freeze-dried).

The problems arising during solubilisation of peptide mixtures are apparent taking in consideration the dissolving behaviour of single peptides. The addition of mild alkali solutions containing e.g. NH₄OH increases the solubility of acidic peptides. In contrast mild acid solutions containing e.g. TFA help to solubilise basic peptides. Water soluble peptides can interact with each other depending on their respective charge state (e. g. oligo-cationic peptides will interact with oligo-anionic peptides to form an insoluble salt that precipitates from aqueous solutions). Highly hydrophobic peptides are regularly dissolved in organic solvents such as DMSO, DMF, acetonitrile, acetic acid or in mixtures of water with the aforementioned solvents. Furthermore it is known to the man skilled in the art that salts tend to promote aggregation of hydrophobic molecules, especially peptides, which is followed by a concentration dependent precipitation. Therefore the solubilisation of a peptide mixture containing different kinds of peptides is a main problem in the manufacturing of e.g. pharmaceutical compositions.

The European patent EP 0 611 572 B1 discloses a method for solubilizing a decapeptide (Cetrorelix) by using an aqueous solution containing 30% acetic acid. Prior freeze-drying this solution is further diluted with water to give a final concentration of acetic acid of 3%. The corresponding US patent application US 2002/0198186 A1 extended the described method to peptides containing 3 to 14 amino acids. However, both the European patent and the US patent application disclose the solubilisation and lyophilisation of single peptides only and not mixtures of two or more peptides. Moreover, it has to be emphasized that dilution of the solution to 3% acetic acid will precipitate hydrophobic peptides resulting in a turbid suspension.

Other commonly used solvents for peptides are dimethyl sulfoxid (DMSO; see e.g. Chen and Wetzel, Protein Science 10:887-891) and dimethylformamide (DMF; see e.g. de Groot et al., Molecular Cancer Therapeutics 1:901-911). The peptides dissolved by these solvents are usually hydrophobic and poorly soluble in water. Unfavorably peptide solutions containing DMSO or DMF can not be lyophilised and can not be used directly for medicinal purposes.

It is the aim of the present invention to provide methods for the solubilisation of peptide mixtures, which are composed of two or more peptides consisting of hydrophilic as well hydrophobic peptides. Another aim is to make available a method for the manufacturing of sterile and optionally lyophilised pharmaceutical formulations containing peptide mixtures.

Therefore, the present invention provides a method for making a pharmaceutical preparation comprising the solubilisation of a peptide mixture, characterized in that the peptide mixture is solubilized by an aqueous solution containing at least one organic acid selected from the group consisting of formic acid, acetic acid, propionic acid, butyric acid and halogenated or hydroxylated forms thereof. The concentration of the organic acid in the aqueous solution is at least 10 %. In order to elongate the shelf life the solubilised peptide mixture may be further sterilised (by filtration, irradiation, heat treatment, chemical sterilisation or other methods) and lyophilised.

In a preferred embodiment the peptide mixture contains hydrophilic as well hydrophobic peptides. Of course the present method is also usable for peptide mixtures containing only one type of peptides (specifically for mixtures containing e.g. two or more hydrophilic peptides).

In another preferred embodiment the peptides which are dissolved by an aqueous solution according to the present invention contain at least 6, preferably at least 8, more preferably at least 10, especially at least 12 amino acids. Peptides as well polypeptides containing a maximum number of 100 amino acids can be dissolved according to the present invention.

In the scope of the present invention peptides are characterised by their solubility. Peptides which are soluble in an aqueous solution less than 100µg/ml are considered hydrophobic. On the other hand hydrophilic peptides dissolve in a buffered aqueous solution in concentration over 100µg/ml. Hydrophilic peptides may further be divided into cationic (>20% basic amino acids including lysine, arginine, histidine), anionic (>20% acidic amino acids including aspartic acid, glutamic acid) and hydroxyl-rich peptides (>30% -OH containing amino acids like serine, threonine, tyrosine).

In another preferred embodiment the peptide mixture contains peptides and/or polypeptides obtained from antigens. Preferably, peptides or polypeptides derived from a viral or a bacterial pathogen or from fungi or parasites are used as such antigens (including derivatized antigens or glycosylated or lipidated antigens or polysaccharides or lipids). Another preferred source of antigens are tumor antigens. Preferred pathogens are selected from human immunodeficiency virus (HIV), hepatitis A and B viruses, hepatitis C virus (HCV), rous sarcoma virus (RSV), Epstein Barr virus (EBV) Influenza virus, Rotavirus, Staphylococcus aureus, Chlamydia pneumonias, Chlamydia trachomatis, Mycobacterium tuberculosis, Streptococcus pneumonias, Bacillus anthracis, Vibrio cholerae, Plasmodium sp. (Pl. falciparum, P1. vivax, etc.), Aspergillus sp. or Candida albicans. Antigens may also be molecules expressed by cancer cells (tumor antigens). The derivation process may include the purification of a specific protein from the pathogen/cancer cells, the inactivation of the pathogen as well as the proteolytic or chemical derivatization or stabilisation of such a protein. In the same way also tumor antigens (cancer vaccines) or autoimmune antigens may be mixed to obtain a composition according to the present invention. With such compositions a tumor vaccination or a treatment for autoimmume diseases may be performed.

In the case of peptide antigens the use of peptide mimitopes/agonists/superagonists/antagonists or peptides changed in certain positions without affecting the immunologic properties or non-peptide mimitopes/agonists/superagonists/antagonists is included in the current invention. Peptide antigens may also contain elongations either at the carboxy or at the amino terminus of the peptide antigen facilitating interaction with a polycationic compound(s) or a immunostimulatory compound(s). For the treatment of autoimmune diseases peptide antagonists may be applied.

Antigens may also be derivatized to include molecules enhancing antigen presentation and targeting of antigens to antigen presenting cells.

A composition obtained by the method according to the present invention, especially in the form of a vaccine, may further comprise a polycationic compound, preferably a polycationic polymer, more preferably a polycationic peptide, especially polyarginine, polylysine or an antimicrobial peptide.

The polycationic compound(s) to be used according to the present invention may be any polycationic compound which shows the characteristic effect according to the WO 97/30721. Preferred polycationic compounds are selected from basic polypeptides, organic polycations, basic polyaminoacids or mixtures thereof. These polyaminoacids should have a chain length of at least 4 amino acid residues. Especially preferred are substances containing peptidic bonds, like polylysine, polyarginine and polypeptides containing more than 20%, especially more than 50% of basic amino acids in a range of more than 8, especially more than 20, amino acid residues or mixtures thereof. Other preferred polycations and their pharmaceutical compositions are described in WO 97/30721 (e.g. polyethyleneimine) and WO 99/38528. Preferably these polypeptides contain between 20 and 500 amino acid residues, especially between 30 and 200 residues. These polycationic compounds may be produced chemically or recombinantly or may be derived from natural sources.

Cationic (poly)peptides may also be polycationic anti-bacterial microbial peptides. These (poly)peptides may be of prokaryotic or animal or plant origin or may be produced chemically or recombinantly. Peptides may also belong to the class of defensins. Such host defense peptides or defensins are also a preferred form of the polycationic polymer according to the present invention. Generally, a compound allowing as an end product activation (or down-regulation) of the adaptive immune system, preferably mediated by APCs (including dendritic cells) is used as polycationic polymer.

Polycationic substances used in the method according to the present invention are cathelicidin derived antimicrobial peptides or derivatives thereof (WO 02/13857), especially antimicrobial peptides derived from mammalian cathelicidins, preferably from human, bovine or mouse, or neuroactive compounds, such as (human) growth hormone (as described e.g. in WO 01/24822).

Polycationic compounds derived from natural sources include HIV-REV or HIV-TAT (derived cationic peptides, antennapedia peptides, chitosan or other derivatives of chitin) or other peptides derived from these peptides or proteins by biochemical or recombinant production. Other preferred polycationic compounds are cathelin or related or derived substances from cathelin, especially mouse, bovine or especially human cathelins and/or cathelicidins. Related or derived cathelin substances contain the whole or parts of the cathelin sequence with at least 15-20 amino acid residues. Derivations may include the substitution or modification of the natural amino acids by amino acids which are not among the 20 standard amino acids. Moreover, further cationic residues may be introduced into such cathelin molecules. These cathelin molecules are preferred to be combined with the antigen/vaccine composition according to the present invention. However, these cathelin molecules surprisingly have turned out to be also effective as an adjuvant for a antigen without the addition of further adjuvants. It is therefore possible to use such cathelin molecules as efficient adjuvants in vaccine formulations with or without further immunactivating substances.

Another preferred polycationic substance to be used according to the present invention is a synthetic peptide containing at least 2 KLK-motifs separated by a linker of 3 to 7 hydrophobic amino acids, especially L (WO 02/32451).

Such peptide mixtures are useful for the formulation of vaccines. Of course also peptide mixtures containing peptides acting as e.g. hormones can be dissolved by the method according to the present invention.

The peptides of the peptide mixture are synthesized by standard chemical methods (e.g. solid phase synthesis according to Merrifield). Of course the peptides may also be obtained by chemical or enzymatic fragmentation of recombinant produced or native isolated proteins. In another embodiment the peptides are directly isolated from eukaryotic and prokaryotic cells. In all three cases additional purification of the peptide or polypeptide of interest will in some cases be required before they are lyophilised and mixed together.

Depending on the application the number of the individual peptides and especially their concentration in the mixture varies. In a preferred embodiment of the present invention the concentration of the individual peptides ranges from 5µg/ml to 5mg/ml, preferably from 50µg/ml to 4mg/ml, more preferably from 100µg/ml to 3mg/ml.

Peptides are often used in pharmaceutical formulations for the use e.g. as hormones or vaccines. Therefore it is a basic requirement that the organic acids used in aqueous solutions for the solubilisation of peptides are pharmaceutically acceptable. In a preferred embodiment of the present invention the organic acids are acetic acid and/or formic acid containing optionally acetonitrile.

The experiments revealed, depending on the composition of the peptide mixture, that the concentration of the organic acid in the aqueous solution has to be at least 10%, preferably at least 20%, preferably at least 30%, preferably at least 40%, preferably at least 50%, preferably at least 60%.

In some cases the addition of derivatives of organic acids (e.g. acetonitrile) to the aqueous solution may be helpful to solubilise peptide mixtures containing a larger quantity of hydrophobic peptides. Also organic solvents such as DMSO and DMF contribute to an enhanced dissolution of peptide mixtures containing an increased concentration of hydrophobic peptides. However, aqueous peptide mixtures containing DMSO and/or DMF cannot be freeze-dried.

In a preferred embodiment bulking agents are added to the peptide mixture when the product is intended to be lyophilised. Especially at low peptide concentrations the forming of a good lyophilisation cake is not guaranteed. Therefore at low amounts of peptides bulking agents are added. Preferred bulking agents are sorbitol, mannitol, polyvinylpyrrolidone (PVP) and mixtures thereof.

In another preferred embodiment the solubilised peptide mixture is sterilised by filtration. In order to get an increased recovery of the product and to allow filtration of large amounts of the peptide mixture, the containing peptides have to be completely solubilised and no gel may be formed.

The solubilised and optionally sterilised peptide mixture can be lyophilised directly or after filling into vials. Lyophilisation is a useful and effective method to prolong the shelf life of peptide mixtures as described above. With the solubilised peptides a lyophilised preparation of a mixture of peptides, containing a maximum of 5% of residual solvent (water in aqueous systems) and traces of the organic acid, can be obtained which is reconstitutable in a buffered aqueous solution containing NaCl and/or sorbitol within 10 minutes of >95%, preferably of 98%, especially of 99%, resulting in a turbid suspension or clear solution (depending on the composition of the peptide mixture). Such a quick reconstitution is specifically necessary in emergency cases, where a ready-to-use solution has to be present within a few minutes with an almost complete re-solvation of the whole dosis of a lyophilised vial.

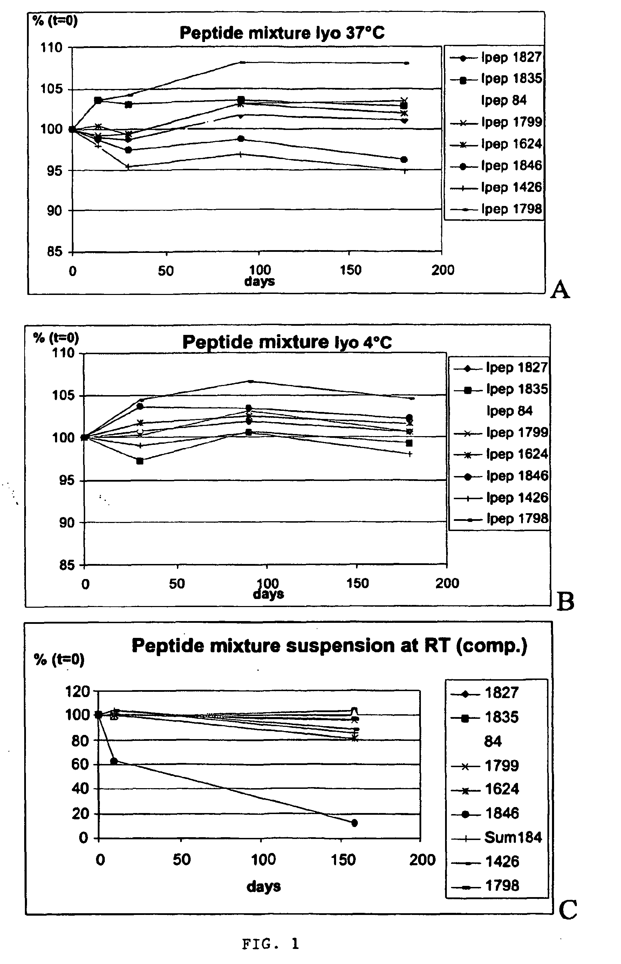

The invention is further illustrated by the following examples and the drawing figure without being restricted to them.

The figure shows the storage stability of a peptide mixture according to the present invention (A, B) compared to suspensions according to the prior art (C).

### Example 1: Freeze-drying of a peptide mixture (5 TB peptides plus poly-L-arginine)

The peptide mixture contains 6 peptides (see table 1), which show a broad range of solubility. Peptides 1240 and 1242 are water soluble and can also be dissolved in DMSO. Peptide 1236 does not dissolve in DMSO, but slowly dissolves in water. Peptides 1235 and 1241 are hydrophobic and only dissolve in DMSO.

Peptides 1236, 1240, and 1242 can sequentially be dissolved in water, provided that the final pH is adjusted to pH 8.5. At lower pH a gel is formed presumably due to charge interactions and/or hydrogen bond formation. This gel can not be sterile filtered as it blocks the filter immediately. However, upon addition of poly-L-arginine (pR) to the aqueous solution of the three peptides a precipitate forms that makes sterile filtration of the solution impossible.

Therefore, to achieve a suspension formulation, three stock solutions have to be prepared: one containing peptides 1236, 1240 and 1242 dissolved in a buffer at pH 8.5, one containing poly-L-arginine (pR) disolved in a NaCl solution and one containing peptides 1235 and 1241 dissolved in DMSO. Each solution can be sterile filtered. The combination of any two of these three solutions leads to the formation of a precipitate, resulting in a turbid suspension. The final formulation obtained by this method can not be sterile filtered and not be freeze-dried when containing 10% DMSO.

It was also tested whether these peptides dissolve in DMSO, DMF, dimethylacetamide, 0,1% formic acid/water, formamide or water/acetonitrile mixtures. However it turned out that none of these regularly used solvents gave a solution that could be sterile filtered; the blocking of the filter showed that considerable amounts of undissolved peptides remained in the solution.

Surprisingly it was found that 50% acetic acid/water is an excellent solvent for this peptide mixture (pR, 1235, 1236, 1240, 1241, and 1242) since it dissolves hydrophilic as well as hydrophobic peptides. In addition, due to being a charged molecule itself, it is also able to dissociate ionic complexes. Thus 50% acetic was able to dissolve the above mentioned mixture at the required concentrations (1 mg/ml each peptide, 2 and 4 mg/ml pR). The solution could be sterile filtered using an acetic acid resistant filter (e.g. hydrophilic teflon). In addition mixtures of water and acetic acid readily freeze and can be freeze-dried using standard lyophilisation cycles.

Very surprisingly the freeze-dried mixture of the five peptides (0,5 mg/vial)and poly-L-arginine (1 mg/vial) showed an excellent stability. Even after 4 months at 37°C, the peptide content was between 96,1% and 100,5% of the original value. In contrast, even an optimized suspension formulation showed considerable degradation after 2 months at 37°C (< 90% recovery for Ipep 1240; <75% recovery of Ipep 1236 and Ipep1241) and numerous degradation peaks (absent in the lyo-formulation) were observed in the chromatograms.

**Table 1 Peptide mixture I**

| ***Peptide ID*** | ***Peptide sequence*** |
|---|---|
| 1235 | IDELKTNSSLLTSILTYHVV |
| 1236 | TGSGAGIAQAAAGTVNI |
| 1240 | VSDLKSSTAVIPGYPVAGQV |
| 1241 | NFLLPDAQAAAAGFASK |
| 1242 | YNINISLPSYYPDQKSLENY |

### Example 2: Solubilisation of peptide mixtures (HCV peptides plus poly-L-arginine):

The peptide mixture contains the peptides 83, 84, 87, 89, 1426 and pR as immunizer (see table 2), wherein peptides 83 and 84 are soluble in water and DMSO, peptides 87 and 89 are poorly water soluble, but dissolve easily in DMSO and peptide 1426 is only soluble in DMSO. As with the formulation of example 1, for the manufacturing of a suspension formulation it is necessary first to dissolve peptides 83 and 84 in an aqueous buffer solution and peptides 87, 89, and 1426 in DMSO. After sterile filtration the solutions can be combined to form the final suspension.

Water/acetonitrile mixtures as well as DMSO and DMF did not work as solvents and resulted in suspensions that could not be sterile filtered due to filter blockage. Surprisingly a 50% acetic acid/water mixture was found to dissolve all components. The obtained peptide solution containing the above mentioned peptides could easily sterile filtered without any filter blocking.

**Table 2 Peptide mixture II**

| ***Peptide ID*** | ***Peptide sequence*** |
|---|---|
| 83 | KFPGGGQIVGGVYLLPRRGPRL |
| 84 | GYKVLVLNPSVAAT |
| 87 | DLMGYIPAV |
| 89 | CINGVCWTV |
| 1426 | HMWNFISGIQYLAGLSTLPGNPA |

The following examples (examples 2.1 to 2.12, tables 3 to 14) illustrate that peptide mixtures containing at least two peptides with variable length and amino acid composition are suitable to be solubilised according to the present invention. Furthermore the tables show the known HLA class I and class II epitopes contained within the peptides.

### Example 2.1:

**Table 3 Peptide mixture III**

| ***Peptide ID*** | ***Peptide sequence*** | ***HLA class I*** | ***HLA class II*** |
|---|---|---|---|
| 1835 | KFPGGGQIVGGVYLLPRRGPRLGVRATRK | A2, A3, B7 | DR11 |
| 87 | DLMGYIPAV | A2 | |
| 1624 | LEDRDRSELSPLLLSTTEW | B60 | DR7 |
| 1846 | DYPYRLWHYPCTVNFTIFKV | A2, A11, Cw7 | DR1, 4, 7, 11 |
| 84 | GYKVLVLNPSVAAT | | DR1, 4, 7, 11 |
| 89 | CINGVCWTV | A2 | |
| 1799 | AAWYELTPAETTVRLR | B35 | DR1, 4 |
| 1547 | YLVAYQATVCARAQAPPPSWD | A2 | DR1, 4, 7, 11 |
| 1827 | TAYSQQTRGLLG | A24 | DR1, 7, 11 |
| 1426 | HMWNFISGIQYLAGLSTLPGNPA | A2 | DR1, 4, 7, 11 |
| 1798 | IGLGKVLVDILAGYGAGVAGALVAFK | A2, A3, A11 | DR1, 4, 7 |
| 1829 | SMSYTWTGALITP | A2, B7 | DR1, 7, 11 |

### Example 2.2:

**Table 4 Peptide mixture IV**

| ***Peptide ID*** | ***Peptide sequence*** | ***HLA class I*** | ***HLA class II*** |
|---|---|---|---|
| 1835 | KFPGGGQIVGGVYLLPRRGPRLGVRATRK | A2, A3, B7 | DR11 |
| 1846 | DYPYRLWHYPCTVNFTIFKV | A2, A11, Cw7 | DR1, 4, 7, 11 |
| 1799 | AAWYELTPAETTVRLR | B35 | DR1, 4 |
| 1827 | TAYSQQTRGLLG | A24 | DR1, 7, 11 |
| 1426 | HMWNFISGIQYLAGLSTLPGNPA | A2 | DR1, 4, 7, 11 |
| 1798 | IGLGKVLVDILAGYGAGVAGALVAFK | A2, A3, A11 | DR1, 4, 7 |
| 1829 | SMSYTWTGALITP | A2, B7 | DR1, 7, 11 |

### Example 2.3:

**Table 5 Peptide mixture V**

| ***Peptide ID*** | ***Peptide sequence*** | ***HLA class I*** | ***HLA class II*** |
|---|---|---|---|
| C134 | TTLLFNILGGWVAAQ | A2 | DR1, 7, 11 |
| 1815 | TVNYTIFKI | A11 | |
| 1006 | MWNFISGIQYLAGLSTLPGN | | |

### Example 2.4:

**Table 6 Peptide mixture VI**

| ***Peptide ID*** | ***Peptide sequence*** | ***HLA class I*** | ***HLA class II*** |
|---|---|---|---|
| 84 | GYKVLVLNPSVAAT | | DR1, 4, 7, 11 |
| 87 | DLMGYIPAV | A2 | |
| 89 | CINGVCWTV | A2 | |
| 1426 | HMWNFISGIQYLAGLSTLPGNPA | A2 | DR1, 4, 7, 11 |

### Example 2.5:

**Table 7 Peptide mixture VII**

| ***Peptide ID*** | ***Peptide sequence*** | ***HLA class I*** | ***HLA class II*** |
|---|---|---|---|
| 1051 | YLLPRRGPRL | A2 | |
| 84 | GYKVLVLNPSVAAT | | DR1, 4, 7, 11 |
| 87 | DLMGYIPAV | A2 | |
| 89 | CINGVCWTV | A2 | |
| 1426 | HMWNFISGIQYLAGLSTLPGNPA | A2 | DR1, 4, 7, 11 |

### Example 2.6:

**Table 8 Peptide mixture VIII**

| ***Peptide ID*** | ***Peptide sequence*** | ***HLA class I*** | ***HLA class II*** |
|---|---|---|---|
| 1006 | MWNFISGIQYLAGLSTLPGN | | |
| 1827EX | GWRLLAPITAYSQQTRGLLGCIV | B8 | |

### Example 2.7:

**Table 9 Peptide mixture IX**

| ***Peptide ID*** | ***Peptide sequence*** | ***HLA class I*** | ***HLA class II*** |
|---|---|---|---|
| 1604 | VVCCSMSYTWTGALITPC | | |
| 83 | KFPGGGQIVGGVYLLPRRGPRL | B8 | |
| A130 | DYPYRLWHYPCTVNF | | |
| B46 | AGAAWYELTPAETTV | | |

### Example 2.8:

**Table 10 Peptide mixture X**

| ***Peptide ID*** | ***Peptide sequence*** | ***HLA class I*** | ***HLA class II*** |
|---|---|---|---|
| B84 | GSIGLGKVLVDILAG | | DR1, 4 |
| B96 | LAGYGAGVAGALVAF | | DR1, 4, 7, 11 |
| 1829 | SMSYTWTGALITP | A2, B7 | DR1, 7, 11 |
| A135 | LWHYPCTVNFTIFKV | | DR7 |
| A130 | DYPYRLWHYPCTVNF | | DR1, 4 |
| 1426 | HMWNFISGIQYLAGLSTLPGNPA | A2 | DR1, 4, 7, 11 |
| 1817 | RMYVGGVEHRL | | DR4 |
| 87EX | DLMGYIPLVGAPL | A2, 24 | |
| 1816 | TINYTIFK | A11 | |

### Example 2.9:

**Table 11 Peptide mixture XI**

| ***Peptide ID*** | ***Peptide sequence*** | ***HLA class I*** | ***HLA class II*** |
|---|---|---|---|
| 1650 | VDYPYRLWHYPCTVNFTIFKVRMYVGGVEHRL | Cw7,A2,A24, A11, A3 | DR1,4,7,11 |
| 1836 | DYPYRLWHYPCTVNFTIFKI | Cw7,A2,A24, A11 | DR1, 4, 7, 11 |
| 1846 | DYPYRLWHYPCTVNFTIFKV | Cw7, A2, A24, A1, A11 | DR1,4,7,11 |
| 1651 | VDYPYRLWHYPCTVNYTIFKIRMYVGGVEHRL | | |
| 1800 | DYPYRLWHYPCTVNYTIFKI | Cw7,A24,A11 | DR7 |

### Example 2.10:

**Table 12 Peptide mixture XII**

| ***Peptide ID*** | ***Peptide sequence*** | ***HLA class I*** | ***BLA class II*** |
|---|---|---|---|
| 1835 | KFPGGGQIVGGVYLLPRRGPRLGVRATRK | A2, A3, B7 | DR11 |
| 83 | KFPGGGQIVGGVYLLPRRGPRL | A2 B7 | DR11 |
| 84EX | AYAAQGYKVLVLNPSVAAT | A24 | DR1,4,7,11 |
| 87EX | DLMGYIPLVGAPL | A2,A24 | |
| 89EX | GEVQWSTATQSFLATCINGVCWTV | A2 | DR 4, 7 |
| 1426 | HMWNFISGIQYLAGLSTLPGNPA | A2 | DR1,4,7,11 |

### Example 2.11:

**Table 13 Peptide mixture XIII**

| ***Peptide ID*** | ***Peptide sequence*** | ***HLA class I*** | ***HLA class II*** |
|---|---|---|---|
| C114 | TAYSQQTRGLLGCIV | A24, B8 | DR1,4,7,11 |
| 1798 | IGLGKVLVDILAGYGAGVAGALVAFK | A2,24,3,11 | DR1,4,7 |
| 1604 | VVCCSMSYTWTGALITPC | A2,A24,B7 | DR1,4,7,11 |
| 1624 | LEDRDRSELSPLLLSTTEW | A1,2,3,26 | DR7 |

### Example 2.12:

**Table 14 Peptide mixture XIV**

| ***Peptide ID*** | ***Peptide sequence*** | ***HLA class I*** | ***HLA class II*** |
|---|---|---|---|
| 1547 | YLVAYQATVCARAQAPPPSWD | A2 | DR1,4,7,11 |
| A1A7 | MSTNPKPQRKTKRNTNR | A11,B08,B27 | |
| A122EX | LINTNGSWHINRTALNCNDSL | A2,2,3,B8 | DR1,4,7,11 |
| A241 | TTILGIGTVLDQAET | A2,A3 | DR1, 4 |
| B8B38 | FDSSVLCECYDAGAAWYE | A1,2,3,26 | |
| C70EX | ARLIVFPDLGVRVCEKMALY | A2,A3,B27 | |
| C92 | AFCSAMYVGDLCGSV | A2,B51 | DR1,4 |
| C97 | GVLFGLAYFSMVGNW | A2,3,26, B2705, 51 | DR1,4,7 |
| C106 | TRVPYFVRAQGLIRA | A3,24,B7,B8,B 2705 | DR1,4,7 |
| C134 | TTLLFNILGGWVAAQ | A2 | DR1, 7,11 |

### Example 2: Stabiliy of peptide mixtures according to the present invention

Another peptide mix containing the following 8 HCV derived peptides was provided:
- Ipep 1827:: TAYSQQTRGLLG
- Ipep 1835:: KFPGGGQIVGGVYLLPRRGPRLGVRATRK
- Ipep 84:: GYKVLVLNPSVAAT
- Ipep 1799:: AAWYELTPAETTVRLR
- Ipep 1624:: LEDRDRSELSPLLLSTTEW
- Ipep 1846:: DYPYRLWHYPCTVNFTIFKV
- Ipep 1426:: HMWNFISGIQYLAGLSTLPGNPA
- Ipep 1798:: IGLGKVLVDILAGYGAGVAGALVAFK

As in the previous examples the mix of the peptides did not dissolve in aqueous solutions. Specifically, Ipep 1798, though water soluble on its own, interacted with the other water soluble peptides Ipep 84 and 1835, which led to precipitation. On top of that, Ipep 1624 most likely ionically interacted with Ipep 1835 and therefore had also to be added to the DMSO solution. Therefore one mix containing Ipep 84, Ipep 1835 and Ipep 1827 in aqueous buffer and one mix containing the other five peptides in DMSO had to be prepared and mixed in a 9:1 ratio (v/v) to give the final suspension. Surprisingly, the whole mix was soluble in 30% and 50% acetic acid and could be freeze-dried from this solution. Even more surprisingly, the final formulation turned out to be stable for at least 6 months even at a temperature as high as 37°C. In fact there was hardly any difference between storage at 5°C and 37°C. In both cases the recovery after 6 months was between 95% and 110% of the initial value (Figure A and B). In contrast, a suspension formulation stored at room temperature (as a comparison to the prior art) showed a higher degree of degradation, mainly oxidation of Ipep 1846 to its dimer (Figure C). However, the recovery of some other peptides (Ipep 1426 and Ipep 1624) were as low as 80% showing the superiority of the lyo-formulation (Figure C as compared to A or B).

## Claims

1. A method for making a pharmaceutical preparation comprising the solubilisation of a peptide mixture, **characterized in that** the peptide mixture is solubilized by an aqueous solution containing at least one organic acid selected from the group consisting of formic acid, acetic acid, propionic acid, butyric acid and halogenated or hydroxylated forms thereof, whereby the concentration of the organic acid in the aqueous solution is at least 10%.

2. The method according to claim 1, **characterized in that** the peptide mixture contains at least three, preferably at least four, especially at least five peptides.

3. The method according to claim 1 or 2, **characterized in that** the peptide mixture contains at least one hydrophilic and/or at least one hydrophobic peptide.

4. The method according to any one of claims 1 to 3, **characterized in that** the peptides contain at least 6, preferably at least 8, more preferably at least 10, especially at least 12 amino acids.

5. The method according to claim 3 or 4, **characterized in that** the hydrophilic peptides are soluble at a concentration of more than 100µg/ml in aqueous solutions.

6. The method according to claim 3 or 4, **characterized in that** the hydrophobic peptides are soluble at a concentration of less than 100µg/ml in aqueous solutions.

7. The method according to any one of claims 1 to 6, **characterized in that** the peptide mixture comprises peptides or polypeptides obtained from bacterial, viral, fungal, parasitic or tumor-associated antigens or fragments thereof.

8. The method according to claim 7, **characterized in that** the antigens are selected from the group consisting of human immunodeficiency virus (HIV), hepatitis A and B viruses, hepatitis C virus (HCV), rous sarcoma virus (RSV), Epstein Barr virus (EBV) Influenza virus, Rotavirus, Staphylococcus aureus, Chlamydia pneumonias, Chlamydia trachomatis, Mycobacterium tuberculosis, Streptococcus pneumonias, Bacillus anthracis, Vibrio cholerae, Plasmodium sp., P1. falciparum, P1. vivax, Aspergillus sp., Candida albicans and tumor antigens.

9. The method according to any one of claims 1 to 8, **characterized in that** the peptide mixture comprises a polycationic compound, preferably a polycationic polymer, more preferably a polycationic peptide, especially, an antimicrobial peptide or a peptide containing at least two KLK-motifs separated by a linker of three to seven hydrophobic amino acids.

10. The method according to any one of claims 1 to 9, **characterized in that** the peptides are chemically synthesised peptides.

11. The method according to any one of claims 1 to 10, **characterized in that** the peptides are obtained by enzymatic or chemical degradation of recombinant or native proteins.

12. The method according to any one of claims 1 to 11, **characterized in that** the peptides are isolated from eukaryotic or prokaryotic organisms.

13. The method according to any one of claims 1 to 12, **characterized in that** the concentration of each solubilised peptide varies from 5µg/ml to 5mg/ml, preferably from 50µg/ml to 4mg/ml, more preferably from 100µg/ml to 3mg/ml.

14. The method according to any one of claims 1 to 13, **characterized in that** the organic acid is a pharmaceutically acceptable organic acid.

15. The method according to any one of claims 1 to 14, **characterized in that** the organic acid is acetic acid and/or formic acid containing optionally acetonitrile.

16. The method according to any one of claims 1 to 15, **characterised in that** the concentration of the organic acid in the aqueous solution is at least 20%, preferably at least 30%, preferably at least 40%, preferably at least 50%, preferably at least 60%.

17. The method according to any one of claims 1 to 16, **characterized in that** the aqueous solution contains additionally at least one derivative of an organic acid, especially acetonitrile.

18. The method according to any one of claims 1 to 17, **characterized in that** the aqueous solution contains additionally at least one organic solvent.

19. The method according to any one of claims 1 to 18, **characterised in that** the aqueous solution contains additionally a bulking agent.

20. The method according to claim 19, **characterised in that** the bulking agent is selected from the group consisting of sorbitol, mannitol, polyvinylpyrrolidone (PVP) and mixtures thereof.

21. The method according to any one of claims 1 to 20, **characterised in that** the solubilised peptide mixture is sterilised by filtration.

22. The method according to any one of claims 1 to 21, **characterised in that** the solubilised peptide mixture is lyophilised and reconstitutable in a buffered aqueous solution containing NaCl and/or sorbitol within 10 minutes of >95%, preferably of 98%, especially of 99%, resulting in a turbid suspension or clear solution.

23. An aqueous solution of a mixture of peptides obtainable with a method according to anyone of claims 1 to 22.

24. The aqueous solution of claim 23, **characterised in that** said solution is a vaccine.

## Patentansprüche

1. Verfahren zur Herstellung eines pharmazeutischen Präparats umfassend die Solubilisierung einer Peptidmischung, **dadurch gekennzeichnet, dass** die Peptidmischung mit einer wässrigen Lösung solubilisiert wird, welche mindestens eine organische Säure ausgewählt aus der Gruppe bestehend aus Ameisensäure, Essigsäure, Propionsäure, Buttersäure und halogenierten oder hydroxylierten Formen davon enthält, wobei die Konzentration der organischen Säure in der wässrigen Lösung mindestens 10% ist.

2. Verfahren nach Anspruch 1, **dadurch gekennzeichnet, dass** die Peptidmischung mindestens drei, vorzugsweise mindestens vier, insbesondere mindestens fünf Peptide enthält.

3. Verfahren nach Anspruch 1 oder 2, **dadurch gekennzeichnet, dass** die Peptidmischung mindestens ein hydrophiles und/oder mindestens ein hydrophobes Peptid enthält.

4. Verfahren nach einem der Ansprüche 1 bis 3, **dadurch gekennzeichnet, dass** die Peptide mindestens 6, vorzugsweise mindestens 8, mehr bevorzugt mindestens 10, insbesondere mindestens 12 Aminosäuren enthalten.

5. Verfahren nach Anspruch 3 oder 4, **dadurch gekennzeichnet, dass** die hydrophilen Peptide bei einer Konzentration von mehr als 100µg/ml in wässrigen Lösungen löslich sind.

6. Verfahren nach Anspruch 3 oder 4, **dadurch gekennzeichnet, dass** die hydrophoben Peptide bei einer Konzentration von weniger als 100µg/ml in wässrigen Lösungen löslich sind.

7. Verfahren nach einem der Ansprüche 1 bis 6, **dadurch gekennzeichnet, dass** die Peptidmischung Peptide oder Polypeptide aufweist, die von bakteriellen, viralen, fungalen, Parasiten- oder Tumor-assoziierten Antigenen oder Fragmenten davon erhalten wurden.

8. Verfahren nach Anspruch 7, **dadurch gekennzeichnet, dass** die Antigene ausgewählt sind aus der Gruppe bestehend aus Humanem Immundefizienz-Virus (HIV), Hepatitis A- und B-Viren, Hepatitis C-Virus (HCV), Rous-Sarcoma-Virus (RSV), Epstein-Barr-Virus (EBV), Influenza-Virus, Rotavirus, Staphylococcus aureus, Chlamydia pneumoniae, Chlamydia trachomatis, mycobacterium tuberculosis, Streptococcus pneumonias, Bacillus anthracis, Bivrio cholerae, Plasmodium sp., Pl. falciparum, Pl. vivax, Aspergillus sp., Candida albicans und Tumor-Antigenen.

9. Verfahren nach einem der Ansprüche 1 bis 8, **dadurch gekennzeichnet, dass** die Peptidmischung eine polykationische Verbindung, vorzugsweise ein polykationisches Polymer, mehr bevorzugt ein polykationisches Peptid, insbesondere ein antimikrobielles Peptid oder ein Peptid, das mindestens zwei durch einen Linker von drei bis sieben hydrophoben Aminosäuren getrennte KLK-Motive enthält, aufweist.

10. Verfahren nach einem der Ansprüche 1 bis 9, **dadurch gekennzeichnet, dass** die Peptide chemisch synthetisierte Peptide sind.

11. Verfahren nach einem der Ansprüche 1 bis 10, **dadurch gekennzeichnet, dass** die Peptide durch enzymatischen oder chemischen Abbau rekombinanter oder nativer Proteine erhalten werden.

12. Verfahren nach einem der Ansprüche 1 bis 11, **dadurch gekennzeichnet, dass** die Peptide aus eukaryontischen oder prokaryontischen Organismen isoliert sind.

13. Verfahren nach einem der Ansprüche 1 bis 12, **dadurch gekennzeichnet, dass** die Konzentration jedes solubilisierten Peptids von 5 µg/ml bis 5 mg/ml, vorzugsweise von 50 µg/ml bis 4 mg/ml, mehr bevorzugt von 100 µg/ml bis 3 mg/ml variiert.

14. Verfahren nach einem der Ansprüche 1 bis 13, **dadurch gekennzeichnet, dass** die organische Säure eine pharmazeutisch akzeptable organische Säure ist.

15. Verfahren nach einem der Ansprüche 1 bis 14, **dadurch gekennzeichnet, dass** die organische Säure Essigsäure und/oder Ameisensäure ist, die gegebenenfalls Acetonitril enthält.

16. Verfahren nach einem der Ansprüche 1 bis 15, **dadurch gekennzeichnet, dass** die Konzentration der organischen Säure in der wässrigen Lösung mindestens 20%, vorzugsweise mindestens 30%, vorzugsweise mindestens 40%, vorzugsweise mindestens 50%, vorzugsweise mindestens 60% ist.

17. Verfahren nach einem der Ansprüche 1 bis 16, **dadurch gekennzeichnet, dass** die wässrige Lösung zusätzlich mindestens ein Derivat einer organischen Säure, insbesondere Acetonitril enthält.

18. Verfahren nach einem der Ansprüche 1 bis 17, **dadurch gekennzeichnet, dass** die wässrige Lösung zusätzlich mindestens ein organisches Lösungsmittel enthält.

19. Verfahren nach einem der Ansprüche 1 bis 18, **dadurch gekennzeichnet, dass** die wässrige Lösung mindestens ein zusätzliches Massemittel enthält.

20. Verfahren nach Anspruch 19, **dadurch gekennzeichnet, dass** das Massemittel ausgewählt ist aus der Gruppe bestehend aus Sorbit, Mannit, Polyvinylpyrrolidon (PVP) und Mischungen davon.

21. Verfahren nach einem der Ansprüche 1 bis 20, **dadurch gekennzeichnet, dass** die solubilisierte Peptidmischung durch Filtration sterilisiert wird.

22. Verfahren nach einem der Ansprüche 1 bis 21, **dadurch gekennzeichnet, dass** die solubilisierte Peptidmischung lyophilisiert wird und in einer gepufferten wässrigen Lösung, enthaltend NaCl und/oder Sorbit, innerhalb von 10 Minuten zu >95%, vorzugsweise 98%, insbesondere 99%, rekonstituierbar ist, was zu einer trüben Suspension oder klaren Lösung führt.

23. Wässrige Lösung einer Mischung von Peptiden, erhältlich mit einem Verfahren nach einem der Ansprüche 1 bis 22.

24. Wässrige Lösung nach Anspruch 23, **dadurch gekennzeichnet, dass** die Lösung ein Vakzin ist.

## Revendications

1. Procédé d'élaboration d'une préparation pharmaceutique comprenant la solubilisation d'un mélange de peptides, **caractérisé en ce que** le mélange de peptides est solubilisé par une solution aqueuse contenant au moins un acide organique choisi dans le groupe constitué par l'acide formique, l'acide acétique, l'acide propionique, l'acide butyrique et les formes halogénées ou hydroxylées de ceux-ci, la concentration de l'acide organique dans la solution aqueuse étant d'au moins 10 %.

2. Procédé selon la revendication 1, **caractérisé en ce que** le mélange de peptides contient au moins trois, de préférence au moins quatre, en particulier au moins cinq peptides.

3. Procédé selon la revendication 1 ou 2, **caractérisé en ce que** le mélange de peptides contient au moins un peptide hydrophile et/ou au moins un peptide hydrophobe.

4. Procédé selon l'une quelconque des revendications 1 à 3, **caractérisé en ce que** les peptides contiennent au moins 6, de préférence au moins 8, plus préférablement au moins 10, en particulier au moins 12 acides aminés.

5. Procédé selon la revendication 3 ou 4, **caractérisé en ce que** les peptides hydrophiles sont solubles à une concentration supérieure à 100 µg/ml dans des solutions aqueuses.

6. Procédé selon la revendication 3 ou 4, **caractérisé en ce que** les peptides hydrophobes sont solubles à une concentration inférieure à 100 µg/ml dans des solutions aqueuses.

7. Procédé selon l'une quelconque des revendications 1 à 6, **caractérisé en ce que** le mélange de peptides comprend des peptides ou des polypeptides obtenus à partir d'antigènes bactériens, viraux, fongiques, parasitaires ou associés à des tumeurs ou de fragments de ceux-ci.

8. Procédé selon la revendication 7, **caractérisé en ce que** les antigènes sont choisis dans le groupe constitué par les antigènes du virus de l'immunodéficience humaine (VIH), des virus de l'hépatite A et B, du virus de l'hépatite C (HCV), du virus du sarcome de Rous (RSV), du virus Epstein Barr (EBV), du virus de la grippe, des rotavirus, de *Staphylococcus aureus*, de *Chlamydia pneumonias*, de *Chlamydia trachomatis,* de *Mycobacterium tuberculosis,* de *Streptococcus pneumonias*, de *Bacillus anthracis*, de *Vibrio cholerae*, de *Plasmodium sp.,* de *Pl. falciparum*, de *Pl. vivax, d'Aspergillus sp. ,* de *Candida albicans*, et les antigènes tumoraux.

9. Procédé selon l'une quelconque des revendications 1 à 8, **caractérisé en ce que** le mélange de peptides comprend un composé polycationique, de préférence un polymère polycationique, plus préférablement un peptide polycationique, en particulier un peptide antimicrobien ou un peptide contenant au moins deux motifs KLK séparés par un lieur de trois à sept acides aminés hydrophobes.

10. Procédé selon l'une quelconque des revendications 1 à 9, **caractérisé en ce que** les peptides sont des peptides synthétisés chimiquement.

11. Procédé selon l'une quelconque des revendications 1 à 10, **caractérisé en ce que** les peptides sont obtenus par dégradation enzymatique ou chimique de protéines recombinantes ou natives.

12. Procédé selon l'une quelconque des revendications 1 à 11, **caractérisé en ce que** les peptides sont isolés à partir d'organismes eucaryotes ou procaryotes.

13. Procédé selon l'une quelconque des revendications 1 à 12, **caractérisé en ce que** la concentration de chaque peptide solubilisé varie de 5 µg/ml à 5 mg/ml, de préférence de 50 µg/ml à 4 mg/ml, plus préférablement de 100 µg/ml à 3 mg/ml.

14. Procédé selon l'une quelconque des revendications 1 à 13, **caractérisé en ce que** l'acide organique est un acide organique pharmaceutiquement acceptable.

15. Procédé selon l'une quelconque des revendications 1 à 14, **caractérisé en ce que** l'acide organique est de l'acide acétique et/ou de l'acide formique contenant éventuellement de l'acétonitrile.

16. Procédé selon l'une quelconque des revendications 1 à 15, **caractérisé en ce que** la concentration de l'acide organique dans la solution aqueuse est d'au moins 20 %, de préférence au moins 30 %, de préférence encore au moins 40 %, plus préférablement au moins 50 %, plus préférablement encore au moins 60 %.

17. Procédé selon l'une quelconque des revendications 1 à 16, **caractérisé en ce que** la solution aqueuse contient également au moins un dérivé d'un acide organique, en particulier de l'acétonitrile.

18. Procédé selon l'une quelconque des revendications 1 à 17, **caractérisé en ce que** la solution aqueuse contient également au moins un solvant organique.

19. Procédé selon l'une quelconque des revendications 1 à 18, **caractérisé en ce que** la solution aqueuse contient également un diluant.

20. Procédé selon la revendication 19, **caractérisé en ce que** le diluant est choisi dans le groupe constitué par le sorbitol, le mannitol, la polyvinylpyrrolidone (PVP) et les mélanges de ceux-ci.

21. Procédé selon l'une quelconque des revendications 1 à 20, **caractérisé en ce que** le mélange de peptides solubilisé est stérilisé par filtration.

22. Procédé selon l'une quelconque des revendications 1 à 21, **caractérisé en ce que** le mélange de peptides solubilisé est lyophilisé et reconstituable dans une solution aqueuse tamponnée contenant du NaCl et/ou du sorbitol en moins de 10 minutes à plus de 95 %, de préférence 98 %, en particulier 99 %, pour obtenir une suspension turbide ou une solution limpide.

23. Solution aqueuse d'un mélange de peptides pouvant être obtenue par un procédé selon l'une quelconque des revendications 1 à 22.

24. Solution aqueuse selon la revendication 23, **caractérisée en ce que** ladite solution est un vaccin.
